(19) Europäisches Patentamt
European Patent Office
Office européen des brevets

(11) Publication number: **0 108 512 B1**

(12) # EUROPEAN PATENT SPECIFICATION

(45) Date of publication of patent specification: **06.12.89**

(51) Int. Cl.[4]: **C 07 C 129/12**, C 07 C 143/74, D 06 M 15/423

(21) Application number: **83306116.1**

(22) Date of filing: **10.10.83**

(54) **Fluoroaliphatic radical-containing, substituted guanidines and fibrous substrates treated therewith.**

(30) Priority: **09.11.82 US 440330**
**09.11.82 US 440317**

(43) Date of publication of application:
**16.05.84 Bulletin 84/20**

(45) Publication of the grant of the patent:
**06.12.89 Bulletin 89/49**

(84) Designated Contracting States:
**CH DE FR GB IT LI NL**

(56) References cited:
**CHEMICAL ABSTRACTS, vol. 97, no. 20, November 15, 1982, page 79, no. 164501t, Columbus, Ohio, US; & JP - A - 82 39 285 (DAINIPPON INK AND CHEMICALS, INC.) 04-03-1982**

(73) Proprietor: **MINNESOTA MINING AND MANUFACTURING COMPANY**
**3M Center, P.O. Box 33427**
**St. Paul, Minnesota 55133 (US)**

(72) Inventor: **Chang, John C. c/o Minnesota Mining and Manufact.Comp. 2501 Hudson Road P.O. Box 33427**
**St. Paul Minnesota 55133 (US)**
Inventor: **Howells, Richard D. c/o Minnesota Mining**
**and Manufact.Comp. 2501 Hudson Road P.O. Box 33427**
**St. Paul Minnesota 55133 (US)**
Inventor: **Williams, Kathryn L. c/o Minnesota Mining**
**and Manufact.Comp. 2501 Hudson Road P.O. Box 33427**
**St. Paul Minnesota 55133 (US)**

(74) Representative: **Baillie, Iain Cameron et al**
**c/o Ladas & Parry Isartorplatz 5**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 108 512 B1

Courier Press, Leamington Spa, England.

**Description**

This invention relates to the treatment of fibrous substrates, such as textile fibers, paper, and leather, with fluorochemical compositions to impart oil and water repellency, and to the resulting treated substrates. In another aspect, it relates to the treatment of carpet fiber with a finish comprising a fluoroaliphatic radical-containing composition to impart oil and water repellency and soil resistance to such fiber. In another aspect, it relates to fluoroaliphatic radical-containing compositions, and their preparation, which are useful in such treatment.

In the industrial production of textiles, such as carpet and apparel, and such other fibrous substrates as paper and leather, it is common to treat such substrates with fluorochemicals containing fluoroaliphatic radicals (often designated by the symbol "$R_f$") to impart oil and water repellency to the surface of such substrates. Fluorochemicals of this type and their application to fibrous substrates are described in various prior art publications, e.g., U.S. Patent Nos. 3,329,661 (Smith et al), 3,458,571 (Tokoli), 3,574,791 (Sherman et al), 3,728,151 (Sherman et al), 3,916,053 (Shearman et al), 4,144,367 (Landucci), 3,896,251 (Landucci), 4,024,178 (Landucci), 4,165,338 (Katsushima et al), 4,215,205 (Landucci), 4,013,627 (Temple), 4,264,484 (Patel), 4,325,857 (Champaneria et al), and Banks, R. E. Ed. "Organofluorine Chemicals and their Industrial Applications", Ellis Horwood, Ltd., West Sussex, England, 226—230 (1979).

Although some fluorochemicals are useful in many applications and many are commercial products, some are relatively expensive to prepare and apply, others are difficult to apply, and others are not durable or do not impart the requirement properties to the extent desired.

Conventionally, fluorochemical compositions have been commercially applied as a top coating to the finished fibrous article, such as carpet. Recently, several fluorochemical compositions have been commercially applied to textile fiber or yarn during its manufacture before it is woven or fabricated into the finished article. However, some of these fluorochemical compositions have had limited success for various reasons including incompatibility or reactivity of the fluorochemical with fiber finish components such as lubricants, lack of durability of the flurorochemical on the treated fiber to dyeing or other fiber manufacturing operations, and insufficient water and oil repellency and soil resistance in the finished article.

It is an object of this invention to provide fluoroaliphatic radical-containing, substituted guanidines (hereinafter often called fluorochemical guanidines for brevity), which optionally can be blended with fluoroaliphatic radical-containing poly(oxyalkylenes) (hereinafter often called fluorochemical oxyalkylenes for brevity) useful for treating textile fibers and other fibrous substrates to impart oil and water repellency thereto.

Another object of this invention is to provide fluorochemical guanidines or blends thereof with fluorochemical oxyalakylenes, which can be used to treat textile fibers in combination with or as a component of fiber finishes, e.g. spin-finish lubricants, such guanidines and blends being compatible with said fiber finishes and not interfering with normal textile fiber processing steps.

A further object of this invention is to provide fluorochemical-treated textile fiber with a high percentage of the fluorochemical retaining on the fiber through fiber processing and dyeing steps, and with durable water and oil repellency and soil resistance properties.

It is yet another object of this invention to provide fluorochemical guanidines and blends thereof with fluorochemical oxyalkylenes, which can be used in the form of organic solutions or aqueous dispersions to treat fibrous substrates such as textile fibers, filaments, yarns, or finished fibrous articles, e.g. carpets, and other fibrous substrates such as paper and leather, to impart oil and water repellency thereto.

The invention provides a normally solid, water-insoluble fluorochemical guanidine compound containing 20 to 70 weight percent carbon-bonded fluorine represented by the general formula:

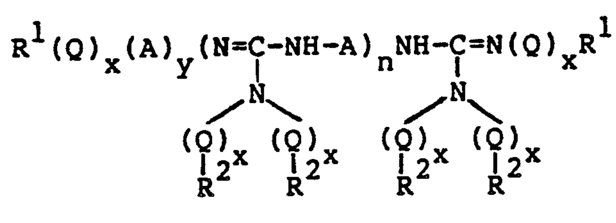

I

and incorporating one or more monovalent fluoroaliphatic radicals, $R_f$, which are both oleophobic and hydrophobic, fluorinated, stable, inert, and non-polar and which have 3 to 20 carbon atoms and at least three fully fluorinated carbon atoms and contains 40 to 78 weight percent fluorine, wherein each x is independently 0 or 1; y is 0 or 1; n is 0 to 20; $R^1$ and $R^2$ are hydrogen atoms said $R_f$, or another organic radical containing 1 to 18 carbon atoms selected from alkyl, cycloalkyl, aryl and combinations thereof which can contain hetero moieties and which are free of active hydrogen atoms, but with the proviso that at least one of $R^1$ and $R^2$ is said $R_f$ and at least one of $R^1$ and $R^2$ is said other organic radical, the two $R^2$ groups of a guanidino moiety,

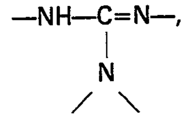

optionally being bonded together to form a cyclic structure incorporating the pendant nitrogen atom,

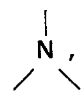

of said guanidino moiety; A is a divalent organic linking group containing 2 to 20 carbon atoms selected from alkylene groups, aralkylene groups, arylene groups, polyoxyaalkylene groups, and combinations thereof, which can contain hetero moieties, which is free of active hydrogen atoms and which may optionally contain said fluoroaliphatic group, $R_f$; Q is a divalent hetero atom-containing or organic linking group, or combination thereof selected from polyvalent aliphatic, polyvalent aromatic, oxy, thio, carbonyl, sulfone, sulfoxy, $—N(CH_3)—$, sulfonamido, carbonamido, sulfonamidoalkylene, carbonamidoalkylene, carbonyloxy, urethane, and urea and which is free of active hydrogen atoms; with the provisos that when only one guanidino moiety is present and only two organic substituents, $—(Q)_xR^1$ or $—(Q)_xR^2$, are in said guanidino moiety, said substituents must be on different nitrogen atoms and that said compound contains 20 to 70 weight percent carbon-bonded fluorine.

Such fluorochemical guanidines are useful in the form of organic solutions or aqueous dispersions in the treatment of fibrous substrates, such as textile fibers (or filaments) during their manufacture, and useful also in the treatment of finished or fabricated fibrous substrates such as carpets, paper, and leather, to impart oil and water repellency to the surface thereof.

This invention also provides compositions comprising blends of two components: (a) said fluorochemical guanidine compositions, and (b) normally liquid or low melting solid, water soluble or dispersible, fluoroaliphatic radical-containing poly(oxyalkylene) compounds, or compositions comprising or consisting essentially of mixtures of said oxyalkylene compounds, which compounds have one or more monovalent fluoroaliphatic radical ($R_f$) and one or more poly(oxyalkylene) moieties, such radicals and oxyalkylene moieties bonded together by hetero atom-containing groups or organic linking groups, or combinations of such groups. Said fluorochemical blends are useful in the form of organic solutions or aqueous dispersions in the treatment of fibrous substrates, such as textile fibers (or filaments) during their manufacture, and useful also in the treatment of finished or fabricated fibrous substrates such as carpets, paper, and leather, to impart oil and water repellency to the surface thereof.

Guanidines are conveniently prepared by the reaction of carbodiimides and imino (NH) compouds, e.g. amines, hydrazines, hydrazides, and amides, using general routes for guanidine synthesis as described, for example, by Kurzer, et al, *Chemical Reviews, 67* 107, (1967), and in U.S. Patent No. 4,174,433 (Schafer, et al). In addition, carbodiimides can be prepared from ureas, thioureas, and other compounds as described by K. Wagner et al., *Angewante Chemie Int. Ed., 20,* 819 (1981). Many fluorochemical guanidines of this invention can be prepared in an analogous manner from fluorochemical carbodiimides and said imino compounds. Such flurochemical carbodiimide and their preparation are described in U.S. Pat. No. 4,024,178 (Landucci), which description is incorporated herein by reference thereto.

In formula I, "n" is a number (in the case where the formula is that of a mixture) or an integer (in the case where the formula is that of a compound) of 0 up to 20, preferably 0 to 10 and most preferably 0 to 5, and "x" is 0 or 1. Each Q is the same or different divalent linking group. A is a divalent organic linking group which can contain a fluoroaliphatic radical, $R_f$, each A being the same of different. Each $R^1$ is the same or different and is selected from H, $R_f$, and terminal monovalent organic radicals such as alkyl, cycloalkyl, aryl, and combinations thereof, e.g. aralkyl, which radicals can contain hetero moieties, e.g.

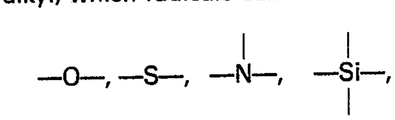

and —CO—, and is free of active (or isocyanate-reactive) hydrogen atoms (i.e., hydrogen atoms of groups, such as mercapto, amino, carboxyl, and aliphatic hydroxyl groups, that can react readily with isocyanate under urethane bond-forming conditions, e.g., 20 to 100°C). Generally, $R^1$ will have no more than 18 carbon atoms. Where $R^1$ is said $R_f$, the subscript x of the adjacent Q must be 1 and not 0 because $R_f$ cannot be directly bonded to a N-atom of the guanidino group. Unless otherwise indicated, "R" means either $R^1$ or $R^2$. Each $R^2$ is like $R^1$ but in addition the two $R^2$ groups of a guanidino group can be bonded together to form a cyclic structure with the adjacent N atom of that guanidino group. There is at least one $R_f$ radical present in one or more of the $R^1$, $R^2$, and A groups for a given compound. When only one guanidino moiety is present, and only two organic substituents are in said guanidino moiety, said substituents must be on different N atoms of the moiety.

In the above general formula I, the divalent organic linking group A connects successive guanidino

moieties when n is 1 or more. Illustrative linking groups A are alkylene groups, such as ethylene, isobutylene, hexylene, and methylenedicyclohexylene, having 2 to 20 carbon atoms, aralkylene groups, such as $—CH_2C_6H_4CH_2—$ and $—C_6H_4CH_2C_6H_4—$, having up to 20 carbon atoms, arylene groups, such as tolylene, $—C_6H_3(CH_3)—$, polyoxyalkylene groups, such as $—(C_2H_4O)_yC_2H_4—$ where y is 1 to about 5, and various combinations of these groups. Such groups can also include other hetero moieties (besides —O—), including —S— and

$$\begin{array}{c}|\\—N—.\end{array}$$

However, A should be free of groups with said active hydrogen atoms.

The A group can be a residue of an organic diisocyanate (from which the carbodiimido and guanidino moieties can be derived by successive reactions), that is, A can be the divalent radical obtained by removal of the isocyanate groups from an organic diisocyanate. Suitable diisocyanate precursors may be simple, e.g. tolylene-2,4-diisocyanate, methylene bis(4-phenyleneisocyanate), and mixtures thereof, or complex, as formed by the reaction of a simple diisocyanate with an organic diol or polyol in appropriate proportions to yield an isocyanate-terminated polyurethane. Other isocyanates can also be used as starting materials. Some of these are described, for example, in U.S. Pat. No. 4,174,433. Representative A groups include $—CH_2C_6H_4CH_2C_6H_4CH_2—$, $—C_6H_3(CH_3)—$, $—C_6H_{10}CH_2C_6H_{10}—$, $—(CH_2)_6—$, $—C_6H_4CH_2C_6H_4—$, and $C_8F_{17}SO_2N[C_2H_4OCONHC_6H_3(CH_3)]_2$. Although the fluorochemical guanidines of this invention generally and preferably are derived from diisocyanates, the fluorochemical guanidines can be derived from triisocyanates, e.g. $OCNC_6H_4CH_2C_6H_3(NCO)CH_2C_6H_4NCO$. A mixture of di- and tri-isocyanates can be used to provide fluorochemical guanidines which are branched but still retain the desired solubility and dispersibility characteristics of the linear fluorochemical guanidines depicted by formula I.

The R—Q groups are preferably radicals derived from isocyanate compounds and can be aliphatic, e.g. $C_6H_{13}—$, aromatic, e.g. $C_6H_5—$, aralkyl, e.g. $C_6H_5CH_2—$, fluoroaliphatic, e.g. $C_6F_{13}CH_2—$, $C_7F_{15}CH_2OCONHC_6H_3(CH_3)—$, and $C_8F_{17}SO_2N(CH_3)C_2H_4OCONHC_6H_4CH_2C_6H_4—$. The organic R—Q radicals can have a variety of other structures, and can contain hetero atom-containing moieties, e.g. —O—, —S—, and

$$\begin{array}{c}|\\—N—,\end{array}$$

but, as with the A group, it is preferably free of groups containing said active hydrogen atoms.

The fluoroaliphatic radical, $R_f$, is a fluoroinated, stable, inert, non-polar, preferably saturated, monovalent moiety which is both oleophobic and hydrophobic. It can be straight chain, branched chain, and, if sufficiently large, cyclic, or combinations thereof, such as alkylcycloaliphatic radicals. The skeletal chain can include catenary oxygen, hexavalent sulfur, and/or trivalent nitrogen herero atoms bonded only to carbon atoms, such hetero atoms providing stable linkages between fluorocarbon portions of $R_f$ and not interfering with the inert character of the $R_f$ radical. $R_f$ should have not more than 20 carbon atoms since large radicals usually represent a less efficient utilization of fluorine than is possible with smaller $R_f$ radicals. The large radicals also are generally less soluble in organic solvents. Generally, $R_f$ will have 3 to 20 carbon atoms, preferably 6 to about 12, and will contain 40 to 78 weight percent, preferably 50 to 78 weight percent, fluorine. The terminal portion of the $R_f$ group has at least three fully fluorinated carbon atoms, e.g. $CF_3CF_2CF_2—$, and the preferred compounds are those in which the $R_f$ group is fully or substantially completely fluorinated, as in the case where $R_f$ is perfluoroalkyl, $C_nF_{2n+1}$.

Generally, the fluorochemical guanidine will contain about 20 to 70 weight percent, preferably about 25 to 50 weight percent, of carbon-bonded fluorine. If the fluorine content is less than about 20 weight percent, impractically large amounts of the fluorochemical guanidine will generally be required, while fluorine contents greater than abut 70 weight percent are unnecessary to achieve the desired surface properties and thus represent an uneconomical use of fluorine and may also present compatibility problems where it is desired to apply the fluorochemical guanidine as an organic solution thereof.

The function of the linking group Q in formula I is to bond the R groups to the N atoms of the guanidino units. Q can comprise a hetero atom-containing group or an organic group or a combination of such groups, examples of which are polyvalent aliphatic, e.g., $—CH_2—$, $—CH_2CH_2—$, and $—CH_2CH(CH_2—)_2$, polyvalent aromatic, oxy, thio, carbonyl, sulfone, sulfoxy, $—N(CH_3)—$, sulfonamido, carbonamido, sulfonamidoalkylene, carbonamidoalkylene, carbonyloxy, urethane, e.g., $—CH_2CH_2OCONH—$, and urea, e.g., —NHCONH—. The linkage Q for a specific fluorochemical guanidine useful in this invention will be dictated by the ease of preparation of such a compound and the availability of necessary precursors thereof. From the above description of Q, it is apparent that this linkage can have a wide variety of structures. However, as with the R and A groups, W should be free of moieties having said active hydrogen atoms. However large Q is, the fluorine content (the locus of which is $R_f$) of the fluorochemical guanidine is in the aforementioned limits.

It should be recognized that, in the above general formula I, isomeric or tautomeric forms may be present. For example, for a given guanidino unit, the following tautomeric forms can exist:

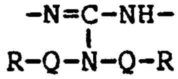

A

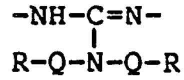

B

When R—Q is H, then another isomeric structure can also be present:

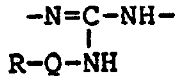

C

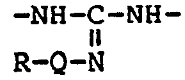

D

All of the above tautomeric and isomeric forms, as well as mixed $R_f$ groups and other organic moieties, can be present and are included in the fluorochemical quanidines of this invention.

The fluorochemical guanidines of this invention are normally solid (i.e., solid at 20°C) with melting points preferably in the range of 40 to 150°C. They are preferably soluble to the extent of at least 10 weight percent in ethyl acetate at 20°C.

The above-described fluorochemical guanidines can be prepared by successive substitutions on guanidine, or by conversion of precursor carbodiimides to guanidines via reaction with imino compounds (i.e., compounds containing >NH), such as primary or secondary amines. The imino compounds may contain a fluoroaliphatic radical in the instance where the carbodiimide precursor contains a fluoroaliphatic radical, and must contain a fluoroaliphatic radical in the instance where the carbodiimide precursor does not contain a fluoroaliphatic radical.

Fluoroaliphatic radical-containing intermediates ($R_f$ intermediates) generally are commercially made by electrochemical fluorination of organic acids or halides thereof, or by telomerization of tetrafluoroethylene, followed by known reactions to form intermediates that contain a hydroxyl group that is capable of reaction with an isocyanate group to form a urethane linkage (—OCONH—). Such urethane-forming reactions are generally carried out neat or in the presence of non-reactive solvents, such as ethyl acetate or methyl ethyl ketone, at moderate temperatures, such as 20 to 130°C. Catalysts for the urethane formation may be employed, but are unnecessary, and in some cases undesirable when aromatic diisocyantes are employed.

The mixture of urethane group-containing isocyanates and non-urethane-containing isocyanates are then converted to the carbodiimide precursors of the fluorochemical guanidines of this invention after addition of low levels (e.g., 0.05 to 1.5 weight percent of reactants) of a catalyst. There are many catalysts known to effect carbodiimide formation from isocyanates. Two of the most effective classes are phospholene oxides (described in U.S. Patent Nos. 2,853,473, 2,941,966, and 4,067,820) and phosphine oxides (described in U.S. Patent No. 3,862,989). The carbodiimide is then added neat or as an organic solvent solution to the imino compound. This mode of addition as well as moderate temperatures are generally employed to minimise the addition of a guanidino N—H moiety to a carbodiimide which generally leads to reaction mixtures that have considerably lower organic solvent solubility.

Representative reaction schemes for the preparation of fluorochemical quanidines of this invention are outlined below, where the product designated as I' are species of formula I supra.

Scheme 1

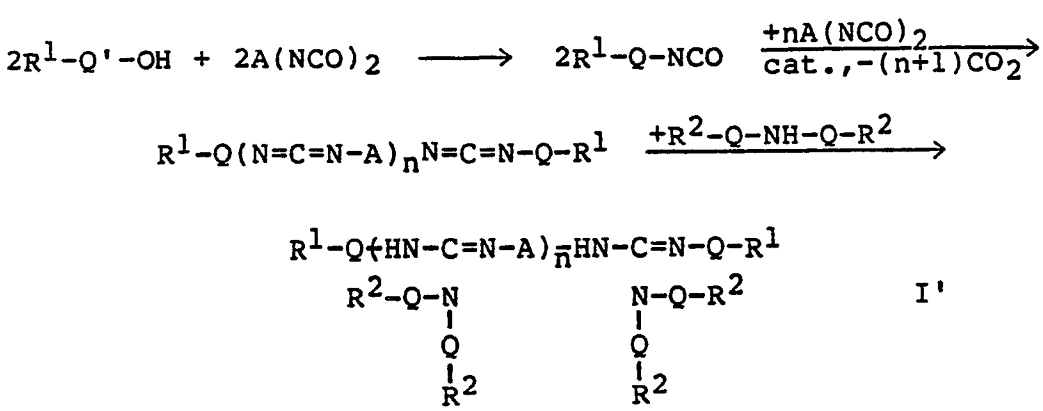


I'

<u>Scheme 2</u>

$$(n+2)A(NCO)_2 \xrightarrow[-(n+1)CO_2]{cat.} OCNA-(N=C=N-A)_nN=C=N-ANCO \xrightarrow{+2R^1-Q'-OH}$$

$$R^1-Q(N=C=N-A)_nN=C=N-Q-R^1 \xrightarrow{+R^2-Q-NH-Q-R^2} I'$$

<u>Scheme 3</u>

$$R-Q'-OH + B(NCO)_3 \longrightarrow R-Q(NCO)_2$$

$$R-Q(NCO)_2 + B(NCO)_3 + A(NCO)_2 + R^1-QNCO \xrightarrow[-CO_2]{cat.} \text{Mixed carbodiimide}$$

The mixtures of fluorochemical guanidines of this invention may contain small amounts of fluorochemical diurethane compounds (e.g., R—Q′—OCONH—A—NHCOO—Q′—R, a possible by-product in Scheme 1) free of quanidino groups due to the synthetic procedures generally followed. The amount of this by-product depends on the mode of addition, molar ratio of reactants, and the relative reactivity of isocyanate functional groups. The mixture of fluorochemical guanidines may contain small or minor amounts of compounds that arise from reaction of an initially formed guanidine with a carbodiimide group to give a higher molecular weight fluorochemical guanidine.

Fluorochemical guanidines in which some of the precursor carbodiimide moieties (in cases where n is greater than 1) have not been reacted with an imino compound are also included as fluorochemical guanidines of this invention.

Representative $R_f$ intermediates for the preparation of fluorochemical guanidines of this inventior include:

$C_8F_{17}SO_2N(C_2H_5)C_2H_4OH$

$C_8F_{17}C_2H_4OH$

$C_7F_{15}CH_2OH$

$C_7F_{15}CON(C_2H_5)C_2H_4OH$

$C_8F_{17}C_2H_4SC_2H_4OH$

$(CF_3)_2CF(CF_2)_8C_2H_4OH$

$(CF_3)_2CFOC_2F_4C_2H_4OH$

$C_8F_{17}C_2H_4SO_2N(CH_3)C_4H_8OH$

$C_8F_{17}SO_2N(CH_3)C_3H_6NH_2$

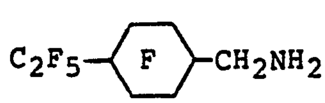

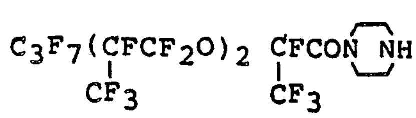

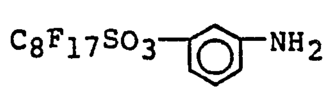

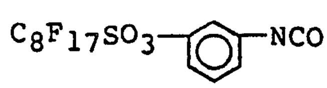

$C_8F_{17}C_6H_4NH_2$

$C_8F_{17}C_6H_4NCO$

$C_7F_{15}CH_2NCO$

Representative organic isocyanates include:

tolylene-2,4-diisocyanate
hexamethylene diisocyanate
methylenebis(4-phenyleneisocyanate)
methylenebis(4-cyclohexyleneisocyanate)
xylylene diisocyanate
1-methoxy-2,4-phenylene diisocyanate
1-chlorophenyl-2,4-diisocyanate
p-(1-isocyanatoethyl)phenyl isocyanate
phenyl isocyanate
m-tolyl isocyanate
2,5-dichlorophenyl isocyanate
hexyl isocyanate

Representative imino compounds include the following: ammonia, methylamine, ethylamine, butylamine, diethylamine, diisopropylamine, dibutylamine, ethyleneimine, morpholine, piperidine, N,N-dimethyl hydrazine, aniline, 3-aminopropyltrimethoxysilane, pyrrolidine, pyrrolidone, imidazole, guanidine, acetamidine, 2-methoxyethylamine, hexamethylenediamine, piperazine, formamide, acetyl hydrazide, sebacoyl dihydrazide.

In cases where certain imino compounds, e.g. ethylene imine, guanidine, N,N'-dialkyl hydrazine, ethylene diamine, and hydrazides are reacted with fluorochemical carbodiimide precursors (Scheme 1, where the above imino compounds are used), adducts are formed which can rearrange to cyclic guanidino structures. These cyclic forms are also included as fluorochemical guanidine compounds of this invention.

A class of fluorochemical oxyalkylene, component (b) — the other essential components of the blends of this invention — are fluoroaliphatic oligomers (or polymers, the term oligomer hereinafter including polymer unless otherwise indicated) represented by the general formulas:

$$(R_f)_sZ[(R^3)_yZ'B]_t \qquad \text{II}$$

$$[(R_f)_sZ[(R^3)_yZ'B']_t]_w \qquad \text{III}$$

where

$R_f$ is a fluoroaliphatic radical like that described for general formula I,

Z is a linkage through which $R_f$ and $(R^3)$y moieties are convalently bonded together,

$(R^3)$y is a poly(oxyalkylene) moiety, $R^3$ being an oxy-alkylene group with 2 to 4 carbon atoms and y is an integer (where the above formulas are those of individual compounds) or a number (where the above formulas are those of mixtures) at least 5, generally 10 to 75 and can be as high as 100 or higher,

B is a hydrogen atom or a monovalent terminal organic radical,

B' is B or a valence bond, with the proviso that at least one B' is a valence bond interconnecting a Z-bonded $R^3$ radical to another Z,

Z' is a linkage through which B, or B', and $R^3$ are covalently bonded together,

s is an integer or number of at least 1 and can be as high as 25 or higher,

t is an integer or number of at least 1, and can be as high as 60 or higher, and

w is an integer or number greater than 1, and can be as high as 30 or higher.

In formulas II and III, where there are a plurality of $R_f$ radicals, they are either the same or different. This also applies to a plurality of Z, Z', $R_3$, B, B', and, in formula III, a plurality of s, y and t.

Generally, the oligomers will contain about 5 to 40 weight percent, preferably about 10 to 30 weight percent, of carbon-bonded fluorine. If the fluorine content is less than about 10 weight percent, impractically large amounts of the oligomer will generally be required, while fluorine contents greater than about 35 weight percent result in oligomers which have too low a solubility to be efficient.

In said poly(oxyalkylene) radical, $(R^3)_y$, $R^3$ is an oxyalkylene group having 2 to 4 carbon atoms, such as

$$-OCH_2CH_2-,\ -OCH_2CH_2CH_2-,$$
$$-OCH(CH_3)CH_2-,\ \text{and}\ -OCH(CH_3)CH(CH_3)-,$$

the oxyalkylene units in said poly(oxyalkylene) being the same, as in poly(oxypropylene), or present as a mixture, as in a heteric straight or branched chain or randomly distributed oxyethylene and oxypropylene units or as in a straight or branched chain of blocks of oxyethylene units and blocks of oxypropylene units. The poly(oxyalkylene) chain can be interrupted by or include one or more catenary linkages. Where said catenary linkages have three or more valences, they provide a means for obtaining a branched chain or oxyalkylene units. The poly(oxyalkylene) radicals in the oligomers can be the same or different, and they

can be pendent. The molecular weight of the poly(oxyalkylene) radical can be about 500 to 2500 and higher, e.g. 100,000 to 200,000 or higher.

The function of the linkages Z and Z′ is to covalently bond the fluoroaliphatic radicals $R_f$, the poly(oxyalkylene) moieties, $(R^3)_y$ and radicals B and B′ together in the oligomer. Z and Z′ can be a valence bond, for example, where a carbon atom of a fluoroaliphatic radical is bonded or linked directly to a carbon atom of the poly(oxyalkylene) moiety. Z and Z′ each can also comprise one or more linking groups such as polyvalent aliphatic and polyvalent aromatic, oxy, thio, carbonyl, sulfone, sulfoxy, phosphoxy, amine, and combinations thereof, such as oxyalkylene, iminoalkylene, iminoarylene, sulfoamido, carbonamido, sulfonamidoalkylene, carbonamidoalkylene, urethane, urea, and ester. The linkages Z and Z′ for a specific oligomer will be dictated by the ease of preparation of such an oligomer and the availability of necessary precursors thereof.

From the above description of Z and Z′ it is apparent that these linkages can have a wide variety of structures, and in fact where either is a valence bond, it doesn't even exist as a structure. However large Z or Z′ is, the fluorine content (the locus of which is $R_f$) is in the aforementioned limits set forth in the above description, and in general the total Z and Z′ content of the oligomer is preferably less than 10 weight percent of the oligomer.

The monovalent terminal organic radical, B, is one which is covalently bonded through Z′, to the poly(oxyalkylene) radical.

Though the nature of B can vary, it preferably is such that it compliments the poly(oxyalkylene) moiety in maintaining or establishing the desired solubility of the oxyalkylene. The radical B can be a hydrogen atom, acyl, such as $C_6H_5C(O)—$, alkyl preferably lower alkyl, such as methyl, hydroxyethyl, hydroxypropyl, mercaptoethyl and aminoethyl or aryl, such as phenyl, chlorophenyl, methoxyphenyl, nonylphenyl, hydroxyphenyl, and aminophenyl. Generally, Z′B will be less than 50 weight percent of the $(R^3)_fZ'B$ moiety.

The fluoroaliphatic radical-containing oxalkylene used in this invention can be prepared by a variety of known methods, such as by condensation, free radical, or ionic homopolymerization or copolymerization using solution, suspension, or bulk polymerization techniques e.g., see "Preparative Methods of Polymer Chemistry," Sorenson and Campbell, 2nd ed., Interscience Publishers, (1968). Classes of representative oxyalkylene useful in this invention include polyesters, polyurethanes, polyepoxides, polyamides and vinyl polymers such as polyacrylates and substitute polystyrenes.

The polyacrylates are a particularly useful class of oxyalkylenes and they can be prepared, for example, by free radical initiated copolymerization of a fluoroaliphatic radical-containing acrylate with a poly(oxyalkylene) acrylate, e.g. monoacrylate or diacrylate or mixtures thereof. As an example, a fluoroaliphatic acrylate, $R_f—R''—O_2C—CH{=}CH_2$ (where R″ is, for example, sulfonamidoalkylene, carbonamidoalkylene, or alkylene), e.g.

$$C_8F_{17}SO_2N(C_4H_9)CH_2CH_2O_2CCH{=}CH_2,$$

can be copolymerized with a poly(oxyalkylene) monoacrylate, $CH_2{=}CHC(O)(R^3)_xOCH_3$, to produce a polyacrylate oxyalkylenes.

Further description of fluorochemical oxyalkylenes useful in this invention will be omitted in the interest of brevity since such compounds and their preparation are known, said U.S. Patent No. 3,787,351 and U.S. Patent No. 4,289,892, both of which are incorporated herein for that purpose.

The amount of each component (a) and (b) can vary over a broad range, and will be selected to provide the desired balance of properties on the treated fiber of finished article. Generally, component (a) will be the major amount of the blend and component (b) will be the minor amount. The particular amount depends on the particular composition of the textile fiber or article to be treated and the particular chemical compositions of (a) and (b), as well as the application procedures used. Laboratory evaluation will often be a good indicator of appropriate relative amounts of components (a) and (b) to be used for obtaining the desired performance in commercial application.

Generally, the relative amounts of components (a) and (b) fall within the following ranges:

| | Weight percent of fluorochemical solids in blend | |
|---|---|---|
| Component | Broad Range | Preferred Range |
| (a) | 60—99 | 70—95 |
| (b) | 1—40 | 5—30 |

The blends of this invention can be obtained by mixing (1) an organic solvent solution or aqueous dispersion of the fluorochemical guanidine with (2) the fluorochemical poly(oxyalkylene) which may be utilized in neat form or as an organic solvent solution or as an aqueous dispersion. If an aqueous emulsion is the desired form of the blend, the emulsification may be performed on the above organic solvent-containing blends, or individually emulsified components may be blended (by simple mixing techniques) as either solvent-containing or sovlent-free emulsions. In the preparation of said emulsions it is generally beneficial to employ cationic fluorochemical surfactants (i.e., $C_8F_{17}SO_2N(H)C_3H_6N(CH)_3Cl$) along with hydrocarbon non-ionic surfactants (i.e., "Tween 80" polyoxyethylene sorbitan monooleate). Since the fluorochemical poly(oxyalkylenes) and mixtures thereof are themselves non-ionic surfactants, the hydrocarbon non-ionic co-surfactants may be totally or partially eliminated by the incorporated of the fluorochemical poly(oxyalkylene) into the solvent-containing blend prior to emulsification.

Substrates which can be treated in accordance with this invention are textile fibers (or filaments), and finished or fabricated fibrous articles such as textiles, e.g. carpet, paper, paperboard, leather, and the like. The textiles include those made from natural fibers, such as cotton and wool, and those made from synthetic organic fibers, such as nylon, acetate, rayon, acrylic, and polyester fibers. Especially good results are obtained on nylon and polyester fibers. The fibers or filaments as such or in an aggregated form, e.g. yarn, tow, web, or roving, or the fabricated textile, e.g., articles such as carpet and woven fabrics, can be treated with the fluorochemical guanidines or blends thereof with the fluorochemical oxyalkylenes. The treatment can be carried out by applying the fluorochemical guanidines or said blends as organic solutions or aqueous or organic dispersions by known techniques customarily used in applying fluorochemicals, e.g. fluorochemical acrylate copolymers, to fibers and fibrous substrates. (If desired, such known fluorochemicals can be used in conjunction with the fluorochemical guanidines or said blends, as will be shown below.) For example, the fluorochemical treatment can be by immersing the fibrous substrates in a bath containing the fluorochemical guanidine or said blend, padding the substrate or spraying the same with the fluorochemical guanidine or said blend, or by foam, kiss-roll, or metering applications, e.g. spin finishing, and then drying the treated substrates if solvent is present. If desired, the fluorochemical guanidine or said blend can be co-applied with conventional fiber treating agents (or adjuvants), e.g. antistatic agents or neat oils (fiber lubricants).

In the manufacture of synthetic organic fibers (see, for example, review articles in Kirk-Othmer, *Encyclopedia of Polymer Science and Technology, 8,* 374—404, 1968), the first step that normally takes place in the process, following initial formation of the filaments (e.g. by melt spinning or solvent spinning), is coating the fiber surface with a small amount (generally less than 2% active solids on fiber) or fiber finish comprising lubricating and antistatic agents. It is particularly advantageous to treat such textile fibers, e.g. nylon 6, with the fluorochemical quanidines or said blend of this invention in conjunction with the spin finish being applied to such textile fibers.

Fiber finishes are generally produced in the form of dilute aqueous emulsions or as an oil ("neat oil") which principally contains said lubricant and antistatic agent as well as emulsifier (surfactant) and may also contain materials such as antioxidants.

Representative lubricants include mineral oils, waxes, vegatable oils (triglycerides) such as coconut oil, peanut oil, and castor oil, synthetic oils, such as esters, polyoxyethylene derivatives of alcohols and acids, and silicone oils.

The antistatic agents, emulsifiers, and surfactants incorporated into the fiber finish are selected from similar chemical classes, which include:

(a) anionics, such as fatty acid soaps, sulfated vegetable oils, salts of alkyl and ethoxylated alkyl phosphates;

(b) cationics, such as fatty amines, quaternary ammonium compounds, and quaternary phosphonium compounds;

(c) nonionics, such as glyceryl monooleate, ethoxylated alcohols, ethoxylated fatty acid, and ethoxylated fatty amides; and

(d) amphoterics, such as betaines, amino acids and their salts.

The preferred mode of applying the fluorochemical guanidines or said blends thereof with fluorochemical oxyalkylenes of this invention to synthetic organic fibers is to incorporate them into the above-described fiber finishes in an amount sufficient to achieve the desired properties, oil and water repellency and soil resistance. Generally, the amount of fluorochemical guanidine or said blend to be used will be that sufficient to retain on the fiber of the finished article, e.g., carpet, about 200 to 1600 ppm fluorine based on the weight of the fiber. Such additions to the conventional fiber finish can be carried out without sacrificing or adversely affecting typical requirements that conventional fiber finishes must meet, namely lubrication, thermal stability, low fuming at elevated temperature, and wetting for fiber dyeability (color addition). The conventional finish components of the fiber finishes containing the fluorochemical guanidines or said blends of this invention are removed in a conventional manner after the fiber is manufactured in fabric form, e.g., carpets and upholstery fabrics. The fluorochemical guanidines and said blends withstand the typical conditions encountered during fiber and yarn processing and also survive the more severe processing conditions which the greige goods encounter, such as scouring and, dyeing, and the finished goods encounter, such as washing, steam cleaning, and dry cleaning. The fluorochemical guanidines and said blends thereof do not interfere with, and are durable through, the normal fiber processing steps, e.g., drawing, texturizing, and heat setting, and provide oil and water repellency and anti-soiling properties to the finished article, e.g., carpet made from the treated fibers.

The conventional application methods used to apply finishes to fibers (or filaments) can be used with the fluorochemical guanidine-containing finishes of this invention. Such methods include the use of either

(a) a revolving ceramic cylinder, i.e., kiss-roll, which is partially immersed in a pan containing the finish, over which the moving filaments pass and pick up a thin film of finish, (b) a metering pump supplying finish through a slot or hole in a fiber guide over which the moving filaments pass, (c) an immersion finish bath, or (d) spraying devices.

The fluorochemical guanidines and blends thereof with fluorochemical oxyalkylenes of this invention are generally compatible with (i.e., dispersible or sufficiently soluble in) commercial neat oil fiber finishes and thus may be mixed with them and coapplied (or applied before or after them). Solubilizing aids, such as "Carbitol" or "Cellosolve" solvents, can be added to the finish to enhance solubility of the fluorochemical guanidines and said blends in the neat oil finish.

Representative fluorochemical guanidines of this invention having the general formula IV are shown in Table 1.

$$R\text{-}Q\text{-}A(NH\text{-}\underset{R^2\text{-}N\text{-}R^2}{\underset{|}{C}}\text{=}N\text{-}A)_n\text{-}Q\text{-}R \qquad IV$$

TABLE 1

| Compound No.* | R—O | A | $NR^2R^2$ |
|---|---|---|---|
| 1 | $C_8F_{17}$—$SO_2N(C_2H_5)C_2H_4OCONH$ | $C_6H_4CH_2C_6H_4$ | $N(C_4H_9)_2$ |
| 2 | $C_8F_{17}$—$SO_2N(C_2H_5)C_2H_4OCONH$ | $C_6H_4CH_2C_6H_4$ | $N(iC_3H_6)_2$ |
| 3 | $C_8F_{17}$—$SO_2N(C_2H_5)C_2H_4OCONH$ | $C_6H_4CH_2C_6H_4$ | $N(C_2H_5)_2$ |
| 4 | $C_8F_{17}$—$SO_2N(C_2H_5)C_2H_4OCONH$ | $C_6H_3(CH_3)$ | $NHCH(CH_3)_2$ |
| 5 | $C_8F_{17}$—$SO_2N(C_2H_5)C_2H_4OCONH$ | $C_6H_3(CH_3)$ | $NHC_{12}H_{25}$ |
| 6 | $C_8F_{17}$—$SO_2N(C_2H_5)C_2H_4OCONH$ | $C_6H_4CH_2C_6H_4$ | 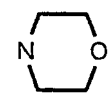 |
| 7 | $C_8F_{17}$—$SO_2N(C_2H_5)C_2H_4OCONH$ | $C_6H_4CH_2C_6H_4$ | $NHN(CH_3)_2$ |
| 8 | $C_8F_{17}$—$SO_2N(C_2H_5)C_2H_4OCONH$ | $C_6H_3(CH_3)$ | $NHC_3H_6Si(OMe)_3$ |
| 9 | $C_8F_{17}$—$SO_2N(C_2H_5)C_2H_4OCONH$ | $C_6H_4CH_2C_6H_4$ | 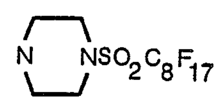 |
| 10 | $(CH_3)_2CHCH_2$—$OCONH$ | $C_6H_4CH_2C_6H_4$ | 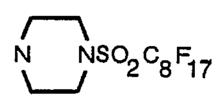 |
| 11 | $C_8F_{17}$—$SO_2N(C_2H_5)C_2H_4OCONH$ | $C_6H_4CH_2C_6H_4$ | 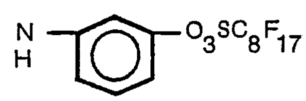 |
| 12 | $C_8F_{17}$—$SO_2N(C_2H_9)C_2H_4OCONH$ | $C_6H_4CH_2C_6H_4$ | $N(C_4H_9)_2$ |
| 13 | $C_8F_{17}$—$C_2H_4OCONH$ | $C_6H_4CH_2C_6H_4$ | $N(C_4H_9)_2$ |
| 14 | $C_8F_{17}$—$C_2H_4OCONH$ | $C_6H_3(CH_3)$ | $N(C_4H_9)_2$ |

* For all compounds listed, n has an average value of 2, except for compound no. 4, where n has a value of about 1.8.

Representative fluorochemical oxyalkylenes useful as component (b) in the fluorochemical blends of this invention are shown in Table 2. Generally the preparation of the fluorochemical oxyalkylenes results in products which comprise mixtures thereof, the length of the fluoroaliphatic radical, and the poly(oxyalkylene) moiety varying and the subscripts denoting the number of carbon atoms of the former and denoting the number of oxyalkylene units in a poly(oxyalkylene) segment being in both cases average

numbers, and in this specification, e.g. Table 2, those subscripts should be understood as having such average values, unless otherwise indicated.

TABLE 2

1. $C_8F_{17}SO_2N(C_2H_5)CH_2CO_2(C_2H_4O)_{15}H$
2. $C_8F_{17}SO_2N(C_2H_5)C_2H_4O(C_2H_4O)_{14}H$
3. $C_8F_{17}C_2H_4O(C_2H_4O)_{15}H$
4. 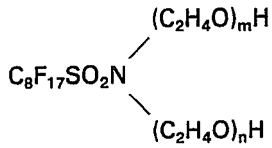 (m+n = 25)
5. $C_8F_{17}SO_2N(C_2H_5)C_2H_4O(C_3H_6O)_8H$
6. 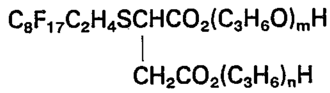 (m+n = 20)

Representative fluorochemical oxyalkylene polyacrylates useful as component (b) in this invention are those made by copolymerizing any of the fluorochemical acrylates of Table 3 with any of the fluorine-free poly(oxyalkylene) monomers of Table 4

TABLE 3

1. $C_8F_{17}SO_2N(CH_3)CH_2CH_2OOCCH{=}CH_2$,
2. $C_6F_{13}C_2H_4OOCC(CH_3){=}CH_2$,
3. $C_6F_{13}C_2H_4SC_2H_4OOCCH{=}CH_2$,
4. $C_8F_{17}C_2H_4OOCC(CH_3){=}CH_2$,
5. $C_8F_{17}C_2H_4N(CH_3)C_2H_4OOCC(CH_3){=}CH_2$,
6. $C_2F_5C_6F_{10}CH_2OOCCH{=}CH_2$,
7. $C_7F_{15}CH_2OOCCH{=}CH_2$,
8. $C_7F_{15}CON(CH_3)C_2H_4OOCCH{=}CH_2$,
9. $(CH_3)_2CF(CF_2)_6CH_2CH_2CH(OH)CH_2OOCCH{-}CH_2{=}CH_2$,
10. $(CH_3)_2CFOC_2F_4C_2H_4OOCCH{=}CH_2$,
11. $C_8F_{17}C_2H_4SO_2N(C_3H_7)C_2H_4OOCCH{=}CH_2$,
12. $C_7F_{15}C_2H_4CONHC_4H_8OOCCH{=}CH_2$,
13. 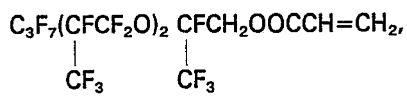
14. $C_7F_{15}COOCH_2C(CH_3)_2CH_2OOCC(CH_3){=}CH_2$,
15. $C_8F_{17}SO_2N(C_2H_5)C_4H_8OOCCH{=}CH_2$,
16. $(C_3F_7)_2C_6H_3SO_2N(CH_3)C_2H_4OOCCH{=}CH_2$,
17. 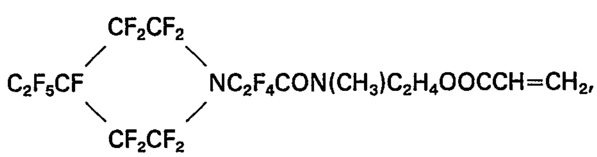
18. $C_6F_{17}CF{=}CHCH_2N(CH_3)C_2H_4OOCCH{=}CH_2$,
19. $C_8F_{17}SO_2N(C_4H_9)C_2H_4OCOCH{=}CH_2$,
20. $C_8F_{17}SO_2N(C_2H_5)C_2H_4OCOCH(CH_3){=}CH_2$,

TABLE 4

1. $CH_2{=}CHCO_2C_2H_4O)_{10}(C_3H_6O)_{22}(C_2H_4O)_9C_2H_4O_2CH{=}CH_2$.
2. $CH_2{=}CHCO_2(C_2H_4O)_{17}H$
3. $CH_2{=}C(CH_3)CONH(C_3H_6)_{44}H$
4. $CH_2{=}C(CH_3)CO_2(C_2H_4O)_{90}COC(CH_3){=}CH_2$,
5. $HS(C_2H_4O)_{23}(C_3H_6O)_{35}(C_2H_4O)_{22}C_2H_4SH$.

Other compatible optional comonomers, e.g. butyl acrylate, acrylonitrile, etc. which need not contain fluoroaliphatic radicals, can be copolymerized with the fluorochemical acrylate and oxyalkylene comonomers in amounts up to above 25 weight percent.

Weight ratios of fluorochemical acrylate monomers (Table 3) and fluorochemical poly(oxyalkylene) monomers (Table 4) can vary but should be chosen along with said optional comonomers so that the carbon-bonded fluorine content of the resulting copolymer is in the desired range of 5 to 40 weight percent.

Objects and advantages of this invention are illustrated in the following examples.

### Example 1

In a 2-liter, 3-neck flask, fitted with a mechanical stirrer, condenser, thermometer, addition funnel and electric heating mantle, was placed 375 g (1.5 moles) methylenebis(4-phenyleneisocyanate) and 481 g methyl ethyl ketone (MEK). To this stirred heated solution (80—83°C) was added 554 g (1.0 mole) N-ethyl(perfluorooctane)sulfonamidoethyl alcohol over a 3 hour period and stirring and heating continued for an additional 3 hours.

To this stirred solution, containing fluorochemical urethane isocyanate and unreacted diisocyanate, was added 7.4 g camphene phenyl phosphine oxide, $C_{10}H_{16}POC_6H_5$, a carbodiimide-forming catalyst, and the reaction mixture was stirred and heated at about 80°C for about 8 hours, at which time essentially all of the isocyanate groups had been converted to carbodiimide groups as indicated by Ir absorption analysis.

The resulting solution of fluorochemical carbodiimide wsa then allowed to cool to room temperature and added over a one hour period to a stirred solution of 129 g (1.0 mole) dibutylamine in 129 g MEK maintained at 30°C. The resulting reaction mixture was heated for one hour at 50°C to complete the conversion of essentially all carbodiimide groups to guanidine groups as indicated by IR analysis. The solid fluorochemical guanidine product (represented by structure 1 in Table 1), isolated in quantitative yield by evaporation of the MEK solvent under reduced pressure, was found to have a melting range of 75—83°C.

### Examples 2—14

Following the general procedure of Example 1, except employing the reagents in Table 5 and molar concentrations indicated in Table 6, the other fluorochemical guanidines of Table 1 were prepared. The reagents in Table 5 are identified by symbols, e.g. A—1, etc., for later reference.

TABLE 5

| | Alcohol Reagents |
|---|---|
| A—1 | $C_8F_{17}SO_2N(C_2H_5)C_2H_4OH$ |
| A—2 | $C_8F_{17}SO_2N(C_4H_9)C_2H_4OH$ |
| A—3 | $C_8F_{17}C_2H_4OH$ |
| A—4 | $(CH_3)_2CHCH_2OH$ |

| | Isocyanates |
|---|---|
| MDI | 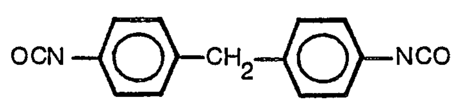 |
| TDI | 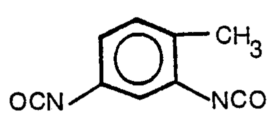 |

Imino Regents

| | |
|---|---|
| I—1 | $(C_4H_9)_2NH$ |
| I—2 | $(iso—C_3H_7)_2NH$ |
| I—3 | $(C_2H_5)_2NH$ |
| I—4 | $(CH_3)_2CHNH_2$ |
| I—5 | $C_{12}H_{25}NH_2$ |
| I—6 | 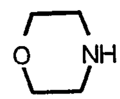 |
| I—7 | $(CH_3)_2NNH_2$ |
| I—8 | $(CH_3O)_3SiC_3H_6NH_2$ |
| I—9 | 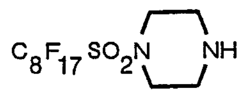 |
| I—10 | 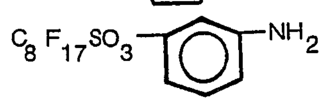 |

TABLE 6

| Ex. No. | Compound used* | Reactants (moles)** Alcohol Reagent | Isocyanate | Imino Reagent |
|---|---|---|---|---|
| 2 | 2 | A—1 | MDI | I—2 |
| 3 | 3 | A—1 | MDI | I—3 |
| 4 | 4 | A—1 (2) | TDI (2.8) | I—4 (1.8) |
| 5 | 5 | A—1 | TDI | I—5 |
| 6 | 6 | A—1 | MDI | I—6 |
| 7 | 7 | A—1 | MDI | I—7 |
| 8 | 8 | A—1 | TDI | I—8 |
| 9 | 9 | A—1 | MDI | I—9 |
| 10 | 10 | A—4 | MDI | I—9 |
| 11 | 11 | A—1 | MDI | I—10 |
| 12 | 12 | A—2 | MDI | I—1 |
| 13 | 13 | A—3 | MDI | I—1 |
| 14 | 14 | A—3 | TDI | I—1 |

* The numbers correspond to the formula numbers of Table 1.
** All alcohol/isocyanate/imino reagent molar ratios were 2/3/2, except as indicated for Example 4.

In the following examples, the above-described fluorochemical guanidines of this invention were used to treat various textile substrates, and the treated articles evaluated for effectiveness of the fluorochemical treatment.

Example 15—28

In these examples, undyed, level loop, nylon 6 carpet was treated in a padding operation (90% wet pickup) with an acetone solution of the fluorochemical guanidine (the concentration of which was in the range of 0.2 to 0.5% solids in order to deposit an amount of fluorochemical equivalent to 700 ppm. fluorine based on the weight of fiber), and 1.7% of a coconut oil based spin finish. The treated carpet was dried for 10 minutes at 50°C. and then heat set at 150°C for 5 min., acid dyed, rinsed, dried (70°C, 30 min.), and cured (heated 130°C, 10 min.).

The oil repellency (OR), water repellency (WR) and walk-on soil resistance (WOS) were determined on the treated samples.

The water repellency test is one which is often used for this purpose. The aqueous stain or water repellency of treated samples is measured using a water/isopropyl alcohol test, and is expressed in terms of a water repellency rating of the treated carpet or fabric. Treated carpets which are penetrated by or resistant only to a 100 percent water/0 percent isopropyl alochol mixture (the least penetrating of the test mixtures) are given a rating of 100/0, whereas treated fabrics resistant to a 0 percent water/100 percent isopropyl alcohol mixture (the most penetrating of the test mixtures) are given a rating of 0/100. Other intermediate values are determined by use of other water/isopropyl alcohol mixtures, in which the percentage amounts of water and isopropyl alcohol are each multiples of 10. The water repellency rating corresponds to the most penetrating mixture with does not penetrate or wet the fabric after 10 seconds contact. In general a water repellency rating of 90/10 or better, e.g., 80/20, is desirable.

The oil repellency test is also one which is often used for this purpose. The oil repellency of treated carpet and textile samples is measured by AATCC Standard Test 118—1978, which test is based on the resistance of treated fabric to penetration by oils of varying surface tensions. Treated fabrics resistant only to "Nujol", a brand of mineral oil and the least penetrating of the test oils, are given a rating of 1, whereas treated fabrics resistant to heptane (the most penetrating of the test oils) are given a value of 8. Other intermediate values are determined by use of other pure oils or mixtures of oils. The rated oil repellency corresponds to the most penetrating oil (or mixture of oils) which does not penetrate or wet the fabric after 10 sec. contact (rather than the 30 sec. contact of the Standard Test). Higher numbers indicate better oil repellency. In general, an oil repellency of 2 or greater is desirable.

The soil resistance of treated and untreated (control) carpet ws determined by exposure to pedestrian traffic in accordance with AATCC Test Method 122—1979, the exposure site being a heavily travelled industrial area for an exosure of about 15,000 "traffics". The samples are repositioned periodically to insure uniform exposure and are vacuumed every 24 hours during the test and before visual evaluation. The evaluation employed the following "Walk-On-Soiling" (WOS) rating system:

| WOS Rating | Description |
|---|---|
| 0 | equal to control |
| $\pm\frac{1}{2}$ | slightly better (+) or worse (−) than control |
| ±1 | impressive difference compared to control |
| $\pm1\frac{1}{2}$ | very impressive difference compared to control |
| ±2 | extremely impressive difference compared to control |

The retention of fluorochemical guanidine on the treated carpet through the dyeing operation was determined by fluorine analysis before and after dyeing.

Results are set sorth in Table 7.

TABLE 7

| Ex. No. | Formula of fluoro-chemical* | Amount Flourine on Carpet, Before Dyeing, ppm | After Dyeing, ppm | Fluorine Retention, Percent | OR | WR | WOS |
|---|---|---|---|---|---|---|---|
| 15 | 1 | 625 | 445 | 71 | 1 | 60/40 | +1½ |
| 16 | 2 | 710 | 415 | 58 | 3 | 40/60 | +2 |
| 17 | 3 | 620 | 440 | 71 | 3 | 50/50 | +½ |
| 18 | 4 | 575 | 575 | 100 | 2 | 70/30 | 0 |
| 19 | 5 | 640 | 350 | 55 | 2 | 70/30 | 0 |
| 20 | 6 | 630 | 555 | 82 | 2 | 60/40 | +1½ |
| 21 | 7 | 665 | 425 | 64 | 2 | 70/30 | +½ |
| 22 | 8 | 525 | 460 | 82 | 1 | 80/20 | 0 |
| 23 | 9 | 720 | 305 | 42 | 1 | 70/30 | 0 |
| 24 | 10 | 545 | 435 | 80 | 1 | 70/30 | 0 |
| 25 | 11 | 775 | 405 | 52 | 2 | 70/30 | +½ |
| 26 | 12 | 680 | 455 | 67 | 2 | 50/50 | +1 |
| 27 | 13 | 585 | 435 | 65 | 2 | 70/30 | +1 |
| 28 | 14 | 750 | 380 | 51 | 2 | 70/30 | 0 |
| C | None | 0 | 0 | 0 | 0 | NWR** | 0 |

* The numbers correspond to formulas of fluorchemical guanidines given in Table 1
** "NWR" means no water repellency

The data of Table 7 shows that oil and water repellency was obtained for all the fluorochemical guanidines used, and soil resistance was obtained for many of them. Particularly noteworthy were the relatively high retention values (after dyeing) which were obtained for most of the fluorochemical guanidines.

Examples 29—32

Two different rainwear fabrics were treated with a 25% aqueous emulsion of the fluorochemical guanidines of formula 6 of Table 1 (using "Tween 80" polyoxyethylene sorbitan monoleate and $C_8F_{17}SO_2NHC_3H_6N(CH_3)_3Cl$ emulsifiers) in a padding operation, dried at 150°C for 10 minutes, and evaluated for initial OR and resistance to a water spray (SR), and these properties evaluated again after 5 launderings (5L) and dry cleaning (DC). The OR test used was the above-described AATCC Standard Test 118—1978, the contact time before observation being the specified 30 sec., an OR value of 3 or greater being particularly desirable.

The water spray rating (SR) is measured by AATCC Test Method 22—1979. The spray rating is measured using a 0 to 100 scale where 100 is the highest possible rating. In general, a spray rating of 70 or greater is desirable, particularly for outwear fabrics.

The treated fabrics were laundered using a mechanically agitated automatic washing machine capable of containing a 4 Kg. load, using water at 50°C and a commercial detergent, and then the washed fabrics were tumble-dried in an automatic dryer for 40 minutes at 70°C and pressed in a flat-bed press (at 154°C) before testing.

The treated fabrics were dry cleaned using perchloroethylene containing 1% of a dry cleaning detergent and tumbling in a motor driven tumble jar (AATCC Test Method 70—1975) for 20 minutes at 25°C. After removing excess solvent in a wringer, samples were dried at 70°C for 10 minutes, then pressed on each side for 15 seconds on a flat-bed press maintained at 154°C.

The runs are summarized in Table 8 together with runs using blends of the fluorochemical guanidine with a comercial fluorochemical used to impart oil and water repellency. Table 8 also includes comparative runs, C—1, C—2, where no fluorochemical was used in a padding operation.

TABLE 8

| Ex. No. | Fluorochemical Used | Fabric[a] | %SOF[b] | Initial OR | Initial SR | 5L OR | 5L SR | DC OR | DC SR |
|---|---|---|---|---|---|---|---|---|---|
| 29 | No. 6 of Table 1 | A | 0.2 | 4.5 | 60 | 3.5 | 70 | 1.5 | 50 |
| 30 | No. 6 of Table 1 | B | 0.2 | 5 | 75 | 3 | 70 | 1.5 | 70 |
| 31 | blend[c] | A | 0.2 | 6.5 | 100 | 5.5 | 75 | 6 | 85 |
| 32 | blend[c] | B | 0.2 | 6.5 | 100 | 4 | 80 | 6.5 | 100 |
| C—1 | —— | A | 0 | 0 | 0 | 0 | 0 | 0 | 0 |
| C—2 | —— | B | 0 | 0 | 0 | 0 | 0 | 0 | 0 |

a. Fabric A was 100% nylon taffeta. Fabric B was 100% woven polyester.
b. % SOF means % fluorochemical solids on fabric.
c. The blend was a mixture of 65 parts of a commercial fluorochemical acrylate copolymer used to treat rainwear and 35 parts of fluorochemical guanindine of formula 6.

The data of Table 8 show useful initial oil and water repellency was obtained for the rainwear fabric when the fluorochemical used was just the fluorochemical guanindine, although laundering and dry cleaning decreased the oil repellency. However, as shown by runs 31, 32, when the blend was used, the repellency did not decrease as much after laundering and dry cleaning.

Example 33

A sample of nylon carpet (50 oz/yd$^2$) was top sprayed with an aqueous emulsion of fluorochemical guanidine (25% aqueous pickup) to impart 0.1% SOF. The treated carpet was dried at 70°C and then cured (heated) at 130°C for 10 min. and found to have OR of 1, WR of 80/20 and, most noteworthy, a WOS of +1-½ to +2.

Example 34

The flourochemical guanidine number 6 of Table 1 was applied as an aqueous dispersion bath at various concentrations to water-leaf paper sheets using a laboratory size press (yielding 82% wet pickup) and the sheets dried in a photo sheet dryer at 150°C and evaluated for oil and water repellency. The results are given in Table 9.

TABLE 9

| Run | Concentration of fluorochemical guanidine in bath wt. % | Amount of fluorochemical on paper, wt. % | Oil repellency[a] | Water repellency[b] |
|---|---|---|---|---|
| 1 | 0.37 | 0.3 | 5 | 64 |
| 2 | 0.61 | 0.5 | 6 | 22 |
| 3 | 1.22 | 1.0 | 8 | 24 |
| 4 | 0 | None | 0 | NWR |

a. This was determined by the "Kit Test" described as TAPPI Useful Method 557; the higher the value the better the repellency.
b. This was determined by the "Cobb Test" descrined as TAPPI—T441—OS—77; the lower the value, the better the water repellency.

The data in the above table show that the fluorochemical guanidine of this invention imparted useful oil repellency, albeit a relatively large amount of the fluorochemical was required. The fluorochemical guanidine may be used in conjunction with hydrocarbon treating agents for paper, e.g. ketene dimers, commonly used to impart the desired water repellency.

Example 35

This example describes the treatment of a carpet fiber with a fluorochemical guanidine of this invention in combination with a spin finish lubricant and the testing of the dyed carpet prepared from the treated fibers.

A neat oil spin finish consisting of 13.1% of the fluorochemical guanidine with formula number 1 of Table 1, 46.2% of a coconut oil-based fiber lubricant, and 40.7% butoxyethoxyethanol was applied to freshly melt-extruded, undrawn yarn of nylon 6 carpet denier fibers. The fluorochemical guanidine was applied with a commercial spin finish applicator. The thus treated yard was continuously drawn and texturized, plied to form a two-ply yarn, heat set at 190°C for one minute, and then made into cut pile carpet. The carpet was acid dyed by three different processes, dried, and then evaluated for oil and water repellency, walk-on soiling resistance, and retention of fluorochemical treatment through the dyeing processes. Control runs were also conducted in the same manner, except that the fluorochemical treatment was omitted; the OR, WR, and WOS values obtained for the control runs were all zero. The testing results are in Table 10.

TABLE 10

| Run | Amount Flourine on Carpet | | Fluorine Retention, Percent | OR | WR | WOS |
|---|---|---|---|---|---|---|
| | Before Dyeing, ppm | After Dyeing, ppm | | | | |
| 1 | 430 | 420[a] | 98 | 2 | 70/30 | 0 |
| 2 | 430 | 400[b] | 93 | 2.5 | 40/60 | +1 |
| 3 | 430 | 350[c] | 81 | 2.5 | 40/60 | 0 |

a. Continuous dye process
b. Beck dye process (batch)
c. Continuous pad dye process

The data of Table 10 shows that outstanding fluorine retention through dyeing and good oil and water repellency were obtained, and better soil resistance was obtained for Beck dyed fiber controls.

Example 36

This example describes the treatment of a nylon 6 carpet fiber with a blend of fluorochemical guanidine and fluorochemical oxyalkylene of this invention dissolved in a spin finish lubricant and the testing of the dyed carpet prepared from the treated fibers.

The oil repellency (OR), water repellency (WR) and walk-on soil resistance (WOS) were determined on the treated samples.

The water repellency test is one which is often used for this purpose. The aqueous stain or water repellency of treated samples is measured using a water/isopropyl alcohol test, and is expressed in terms of a water repellency rating of the treated carper or fabric. Treated carpets which are penetrated by a resistant only to a 100 percent water/0 percent isopropyl alcohol mixture (the least penetrating of the test mixtures) are given a rating of 100/0, whereas treated fabrics resistant to a 0 percent water/100 percent isopropyl alcohol mixture (the most penetrating of the test mixtures) are given a rating of 0/100. Other intermediate values are determined by use of other water/isopropyl alcohol mixtures, in which the percentage amounts of water and isopropyl alcohol are each multiples of 10. The water repellency rating corresponds to the most penetrating mixture which does not penetrate or wet the fabric after 10 seconds contact. In general a water repellency rating of 90/10 or better, e.g. 80/20, is desirable.

The oil repellency test is also one which is often used for this purpose. The oil repellency of treated carpet and textile samples is measured by AATCC Standard Test 118—1978, which test is based on the resistance of treated fabric to penetration by oils of varying surface tensions. Treated fabrics resistant only to "Nujol", a brand of mineral oil and the least penetrating of the test oils, are given a rating of 1, whereas treated fabrics resistant to heptane (the most penetrating of the test oils) are given the value of 8. Other intermediate values are determined by use of other pure oils or mixtures of oil. The rated oil repellency corresponds to the most poenetrating oil (or mixtures of oils) which does not penetrate or wet the fabric

after 10 seconds contact rather than the 30 seconds contact of the Standard Test. Higher numbers indicate better oil repellency. In general, an oil repellency of 2 or greater is desirable.

The soil resistance of treated and untreated (control) carpet was determined by exposure to pedestrian traffic according to AATCC Test Method 122—1979, the exposure site being a heavily travelled industrial area for an exposure of about 15,000 "traffics". The samples are repositioned periodically to insure uniform exposure and are vacuumed every 24 hours during the test and before visual evaluation. The evaluation employed the following "Walk-On-Soiling" (WOS) rating system:

| WOS Rating | Description |
|---|---|
| 0 | equal to control |
| ±½ | slightly better (+) or worse (−) than control |
| ±1 | impressive difference compared to control |
| ±1½ | very impressive difference compared to control |
| ±2 | extremely impressive difference compared to control |

A neat oil spin finish consisting of 13.1% fluorochemical guanidine of formula 1 of Table 1, 5.0% fluorochemical poly(oxyalkylene)/acrylate copolymer (a 30/30/40 terpolymer of $C_8F_{17}SO_2N(C_4H_9)C_2H_4OCOCH=CH_2$, $CH_2=CHCO_2(CH_2H_4O)_{10}(C_3H_6O)_{22}(C_2H_4O)_9C_2H_4O_2CCH=CH_2$, and $CH_2=CHCO_2(C_2H_4O)_{10}(C_3H_6O)_{22}(C_2H_4O)_9C_2H_4OH$, 46.2% of a coconut oil-based fiber lubricant, and 35.7% butoxyethoxyethanol was applied by a metered slot applicator to freshly melt-extruded, undrawn yarn of nylon 6 carpet denier fibers. The thus treated yarn was continuously drawn and texturized, plied to form a two-ply yarn, heat set at 190°C for one minute, and then made into cut pile carpet. The carpet was acid dyed by three different processes, dried, and then evaluated for oil and water repellency, walk-on-soil resistance, and retention of fluorochemical treatment (as measured by fluorine analysis; through the dyeing process. The runs are summarized and the testing results are in Table 11, Runs 1—3. Comparative Runs, C1—C3, utilized a spin finish of the same composition except that the fluorochemical poly(oxyalkylene) component was omitted and 5.0% additional butoxyethoxyethanol was added instead.

TABLE 11

| | Amount Flourine on Carpet | | | | | |
|---|---|---|---|---|---|---|
| Run | Before Dyeing, ppm | After Dyeing, ppm | Retention of Fluorine, % | OR | WR | WOS |
| 1 | 425 | 345[a] | 81 | 3.5 | 50/50 | +1½ |
| 2 | 425 | 335[b] | 79 | 3 | 50/50 | +1 |
| 3 | 425 | 335[c] | 77 | 4 | 50/50 | +1½ |
| C—1 | 430 | 420[a] | 98 | 2 | 70/30 | 0 |
| C—2 | 430 | 400[b] | 93 | 2.5 | 40/60 | +1 |
| C—3 | 430 | 350[c] | 81 | 2.5 | 40/60 | 0 |

a. Continuous dye process was used for dyeing.
b. Beck dye (batch) process was used for dyeing.
c. Continuous pad dye process was used for dyeing.

The test results show that the fluorochemical blend of this invention, Runs 1—3, imparted desirable oil and water repellency and soil resistance to the nylon fiber and the fluorochemical was retained at high levels through dyeing. The results also show significantly better OR and WOS values for Runs 1—3 compared to Runs C—1, C—2, C—3 (even at the lower fluorochemical retention level on fiber), demonstrating the value of the fluorochemical poly(oxyalkylene) component in the finish of this invention.

Example 37

In this invention, two different rainwear fabrics were treated by a padding operation in Runs 1, 2 with an aqueous dispersion of a blend of 20 parts of the fluorochemical guanidine of formula 6 of Table 1 and 1 part of the 30/30/40 terpolymer of Example 36. An aqueous dispersion of said fluorochemical quanidine was used in comparative Runs C—1 and C—2. The treated fabrics were dried at 150°C for 10 minutes and evaluated for initial OR and resistance to water spray (SR) and these properties evaluated again after 5 launderings (5L) and dry cleaning (DC), using the laundering, dry cleaning, and test procedures described in Examples 29—32.

The runs are summarized and the test results are given in Table 12.

TABLE 12

| Run | Fluorochemical Used | Fabric[a] | %SIB[c] | %SOF[b] | Initial | | 5L | | DC | |
|---|---|---|---|---|---|---|---|---|---|---|
| | | | | | OR | SR | OR | SR | OR | SR |
| 1 | Blend | A | 1.01 | 0.21 | 6 | 60 | 4 | 60 | 2 | 50 |
| 2 | Blend | B | 0.23 | 0.21 | 5 | 70 | 4 | 70 | 2 | 70 |
| C—1 | FC—G 6* | A | 0.96 | 0.20 | 4.5 | 60 | 3.5 | 70 | 1.5 | 50 |
| C—2 | FC—G 6* | B | 0.22 | 0.20 | 5 | 75 | 3 | 70 | 1.5 | 70 |
| C—3 | None | A | 0 | 0 | 0 | 0 | 0 | 0 | 0 | 0 |
| C—4 | None | B | 0 | 0 | 0 | 0 | 0 | 0 | 0 | 0 |

* FC—G 6 is the fluorochemical guanidine having formula 6 of Table 1.
a. Fabric A was 100% nylon taffeta. Fabric B was 100% woven polyester.
b. %SOF means % fluorochemical solids on fabric.
c. %SIB means % fluorochemical solids in bath.

The data of Table 12 shows useful oil and water repellency was obtained for the rainwear, though laundering and dry cleaning decreased the oil repellency. Furthermore, the oil and water repellency after laundering or dry cleaning of the fabrics treated with the blend (Runs 1, 2) is better than that of the fabric treated with just the fluorochemical guanidine (Runs C—1, C—2).

Various modifications and alterations of this invention will become apparent to those skilled in the art without departing from the scope of this invention.

## Claims

1. A normally solid, water-insoluble guanidine compound of the general formula:

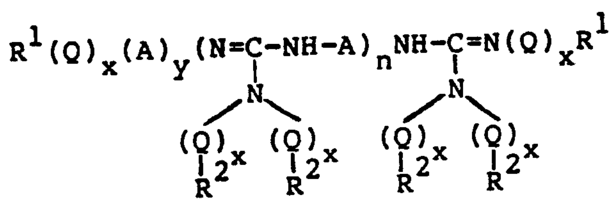

and incorporating one or more monovalent fluoroaliphatic radicals, $R_f$, which are both oleophobic and hydrophobic, fluorinated, stable, inert, and non-polar and which have 3 to 20 carbon atoms and at least three fully fluorinated carbon atoms and at least three fully fluorinated carbon atoms and contains 40 to 78 weight percent fluorine, wherein each x is independently 0 or 1; y is 0 or 1; n is 0 to 20; $R^1$ and $R^2$ are hydrogen atoms, said $R_f$, or another organic radical containing 1 to 18 carbon atoms selected from alkyl, cycloalkyl, aryl and combinations thereof which can contain hereto moieties and which are free of active hydrogen atoms, but with the proviso that at least one of $R^1$ and $R^2$ is said $R_f$ and at least one of $R^1$ and $R^2$ is said other organic radicals, the two $R^2$ groups of guanidino moiety,

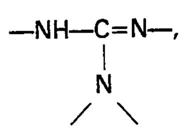

optionally being bonded together to form a cyclic structure incorporating the pendant nitrogen atom,

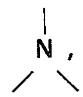

of said quanidino moiety; A is a divalent organic linking group containing 2 to 20 carbon atoms selected from alkylene groups, aralkylene groups, arylene groups, polyoxyalkylene groups, and combinations thereof, which can contain hetero moieties, which is free of active hydrogen atoms and which may optionally contain said fluoroaliphatic group, $R_f$; Q is a divalent hetero atom-containing or organic linking group, or combination thereof selected from polyvalent aliphatic, polyvalent aromatic, oxy, thio, carbonyl, sulfone, sulfoxy, —$N(CH_3)$—, sulfonamido, carbonamido, sulfonamidoalkylene, carbonamidoalkylene, carbonyloxy, urethane, and urea and which is free of active hydrogen atoms; with the provisos that when only one guanidino moiety is present and only two organic substituents, —$(Q)_xR^1$ or —$(Q)_xR^2$, are in said guanidino moiety, said substituents must be on different nitrogen atoms and that said compound contains 20 to 70 weight percent carbon-bonded fluorine.

2. A fluorochemical guanidine according to claim 1 represented by the formula

$$R\text{-}Q\text{-}A(NH\text{-}\underset{\underset{R^2\text{-}N\text{-}R^2}{|}}{C}{=}N\text{-}A)_n\text{-}Q\text{-}R$$

where R—Q is $C_8F_{17}SO_2N(C_2H_5)C_2H_4OCONH$—, A is —$C_6H_4CH_2C_6H_4$— or —$CH_2C_6H_4CH_2$—, $R^2$—N—$R^2$ is —$N(C_4H_9)_2$, and n is 2.

3. A fluorochemical guanidine according to claim 1 represented by the formula

$$R\text{-}Q\text{-}A(NH\text{-}\underset{\underset{R^2\text{-}N\text{-}R^2}{|}}{C}{=}N\text{-}A)_n\text{-}Q\text{-}R$$

where R—Q is $C_8F_{17}SO_2N(C_2H_5)C_2H_4OCONH$—, A is —$C_6H_4CH_2C_6H_4$— or —$CH_2C_6H_4CH_2$—, $R^2$—N—$R^2$ is

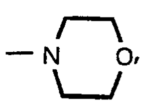, and n is 1 to 21.

4. A fluorochemical guanidine according to claim 1 represented by the formula

$$R\text{-}Q\text{-}A(NH\text{-}\underset{\underset{R^2\text{-}N\text{-}R^2}{|}}{C}{=}N\text{-}A)_n\text{-}Q\text{-}R$$

where R—Q is $C_8F_{17}SO_2N(C_2H_5)C_2H_4OCONH$—, A is —$C_6H_4CH_2C_6H_4$— or —$CH_2C_6H_4CH_2$—, $R^2$—N—$R^2$ is —N(iso—$C_3H_7)_2$, and n is 1 to 21.

5. A composition comprising a blend of: (a) a fluorochemical guanidine composition of any of claims 1—4; and (b) a normally liquid or low melting solid, water soluble or dispersible, fluoroaliphatic radical-containing poly(oxyalkylene), or composition comprising a mixture of such poly(oxyalkylenes), said poly(oxyalkylene) having one or more of said fluoroaliphatic radicals and one or more poly(oxyalkylene) moieties, said radicals and poly(oxyalkylene) moieties bonded together by hereto atom-containing groups or organic linking groups or combinations of said groups.

6. A composition according to claim 5 wherein said fluoroaliphatic radical-containing poly(oxyalkylene) has the general formula

$$(R_f)_sZ[(R^3)_yZ'B]_t \text{ or } [(R_f)_sZ[(R^3)_yZ'B']_t]_w$$

where

$R_f$ is said fluoroaliphatic radical,

Z is a linkage through which $R_f$ and $(R^3)y$ are covalently bonded together,

$(R^3)_y$ is a poly(oxyalkylene) moiety, $R^3$ being an oxyalkylene with 2 to 4 carbon atoms and y is an integer or number of at least 5 and can be as high as 100 or higher,

B is a monovalent terminal organic radical,

B' is B or a valence bond, with the proviso that at least one B' is a valence bond interconnecting a Z-bonded $(R^3)_y$ radical to another Z,

Z' is a linkage through which B, or B' and $(R^3)_y$ are covalently bonded together,

s is an integer or number of at least 1 and can be as high as 25 or higher,

t is an integer or number of at least 1 and can be as high as 60 or higher, and

w is an integer or number greater than 1 and can be as high as 30 or higher.

7. A composition according to claim 5 wherein said fluorochemical poly(oxyalkylene) is the copolymer of

$C_8F_{17}SO_2N(C_4H_9)C_2H_4O_2CCH{=}CH_2$, $CH_2{=}CHCO_2(C_2H_4O)_{10}(C_3H_6O)_{22}(C_2H_4)_9C_2H_4O_2CCH{=}CH_2$, and $CH_2{=}CHCO_2(C_2H_4O)_{10}(C_3H_6O)_{22}(C_2H_4)_9C_2H_4OH$.

8. A composition according to claim 5 wherein said fluorochemical guanidine is represented by the formula

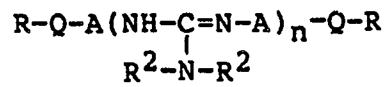 IV

where R—Q is $C_8F_{17}SO_2N(C_2H_5)C_2H_4OCONH$—, A is —$C_6H_4CH_2C_6H_4$— or —$CH_2C_6H_4CH_2$—, $R^2$—N—$R^2$ is $N(C_4H_9)_2$, — 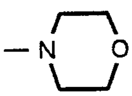 , or —N(iso-$C_3H_7)_2$, and n is 2, and wherein said fluorochemical poly(oxyalkylene) is the copolymer of

$C_8F_{17}SO_2N(C_4H_9)C_2H_4O_2CCH{=}CH_2$, $CH_2{=}CHCO_2(C_2H_4O)_{10}(C_3H_6O)_{22}(C_2H_4O)_9C_2H_4O_2CCH{=}CH_2$, and $CH_2{=}CHCO_2(C_2H_4O)_{10}(C_3H_6O)_{22}(C_2H_4O)_9C_2H_4OH$.

9. A fiber finish comprising an organic solution or aqueous dispersion of a fluorochemical guanidine compound as claimed in any preceding claim.

10. The fiber finish according to claim 9 further comprising a fiber lubricant.

11. A method for imparting oil and water repellency to a fibrous substrate, which comprises treating the surface thereof with a fiber finish as claimed in claim 9 or claim 10.

12. A method of treating spun synthetic organic fibers wherein a fiber finish as claimed in claim 9 or 10 is applied to said fibers.

13. A fibrous substrate coated with a fluorochemical guanidine compound as claimed in any of claims 1 to 8.

14. A coated fibrous substrate as claimed in claim 13 wherein said substrate is nylon carpet fiber.

**Patentansprüche**

1. Normalerweise feste, wasserunlösliche Guanidinverbindung der allgemeinen Formel

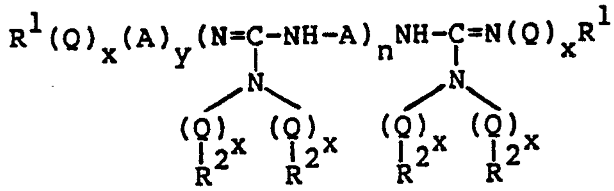

die ein oder mehrere einwertige fluoraliphatische Radikale R enthält, die sowohl oleophob als auch hydrophob sind, und die fluoriert, stabil, inert und nichtpolar ist, 3 bis 20 Kohlenstoffatome enthält, mindestens drei vollständig fluorierte Kohlenstoffatome enthält und 40 bis 78 Gewichtsprozent Fluor enthält, wobei jedes x unabhängig von den anderen gleich 0 oder 1 ist, y gleich 0 oder 1 ist, n gleich 0 bis 20 ist, $R^1$ und $R^2$ Wasserstoffatome sind oder das genannte $R_f$ oder ein anderes 1 bis 18 Kohlenstoffatome enthaltendes, organisches Radikal ist, das aus Alkyl, Cycloalkyl, Aryl und Kombinationen derselben ausgewählt ist, die Heteroanteile enthalten können und frei sind von aktiven Wasserstoffatomen, wobei jedoch mindestens einer der Anteile $R^1$ und $R^2$ das genannte $R_f$ und mindestens einer der Anteile $R_1$ und $R_2$ das genannte andere organische Radikal ist, wobei die beiden $R^2$-Gruppen eines Guanidinoanteils

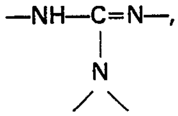, gegebenenfalls miteinander

unter Bildung einer zyklischen Struktur verbunden sind, die das angehängte Stickstoffatom

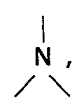,

des Guanidinoanteils enthält; A eine 2 bis 20 Kohlenstoffatome enthaltende, zweiwertige organische Verbindungsgruppe ist, die aus den Alkylen, Aralkylen-, Arylen- und Polyoyalkylengruppen und den Konbinationen derselben ausgewählt ist, die Heteroanteile enthalten können, und die frei ist von aktiven Wasserstoffatomen und die gegebenenfalls die fluoraliphatische Gruppe $R_f$ enthalten kann, Q eine heteroatomhaltige oder organische, zweiwertige Verbindungsgruppe oder eine Kombination derartiger Gruppen ist, die ausgewählt ist aus den mehrwertigen aliphatischen Gruppen, den mehrwertigen

aromatischen Gruppen der Oxy-, Thio-, Carbonyl-, Sulfon-, und Sulfoxygruppe, der Gruppe $—N(CH_3)-$, der Sulfonamido, Carbonamido, Sulfonamidoalkylen-, Carbonamidoalkylen-, Carbonyloxy, Urethan-, und Harnstoffgruppe und die frei ist von aktiven Wasserstoffatomen, wobei, wenn nur ein Guanidinoanteil vorhanden ist und dieser nur zwei organischen Substituenten $—(Q)_xR^1$ oder $—(Q)_xR^2$ enthält, diese Substituenten an verschiedenen Stickstoffatomen angelagert sein müssen und die Verbindung 20 bis 70 Gew.% kohlenstoffgebundenes Fluor enthält.

2. Fluorchemisches Guanidin nach Anspruch 1 der Formel

$$R-Q-A(NH-\underset{\substack{|\\ R^2-N-R^2}}{C}=N-A)_n-Q-R$$

in der R—Q $C_8F_{17}SO_2N(C_2H_5)C_2H_4OCONH—$ ist, A $—C_6H_4CH_2C_6H_4—$ oder $—CH_2C_6H_4CH_2—$ ist, $R^2—N—R^2$. $—N(C_4H_9)_2$ ist und n gleich 2 ist.

3. Fluorchemisches Guanidin nach Anspruch 1 der Formel

$$R-Q-A(NH-\underset{\substack{|\\ R^2-N-R^2}}{C}=N-A)_n-Q-R$$

in der R—Q is $C_8F_{17}SO_2N(C_2H_5)C_2H_4OCONH—$ ist, A $—C_6H_4CH_2C_6H_4—$ oder $—CH_2C_6H_4CH_2—$ ist, $R^2—N—R^2$

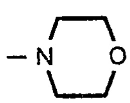 ist und N 1 bis 21 ist.

4. Fluorchemisches Guanidin nach Anspruch 1 der Formel

$$R-Q-A(NH-\underset{\substack{|\\ R^2-N-R^2}}{C}=N-A)_n-Q-R$$

in der R—Q $C_8F_{17}SO_2N(C_2H_5)C_2H_4OCONH—$ ist, A $—C_6H_4CH_2C_6H_4—$ oder $—CH_2C_6H_4CH_2—$ ist, $R^2—N—R^2—N(iso—C_3H_7)_2$ ist und n gleich 1 bis 21 ist.

5. Zusammensetzung mit einem Gemisch aus (a) einer fluorchemischen Guanidinzusammensetzung nach einem der Ansprüche 1 bis 4 und (b) einem normalerweise flüssigen oder niedrigschmelzenden festen, wasserlöslichen oder in Wasser dispergierbaren, ein fluoraliphatisches Radikal enthaltenden Poly(oxyalkylen) oder einer Zusammensetzung die ein Gemisch derartiger Poly(oxyalkylene) enthält, wobei das Poly(oxyalkylen) eines oder mehrere der fluoraliphatischen Radikale und einen oder mehrere Poly(oxyalkylen)anteile enthält und die gennannten Radikale und Poly(oxyalkylen)anteile miteinander durch heteroatomhaltige Gruppen oder organische Verbindungsgruppen oder Kombinationen dieser Gruppen verbunden sind.

6. Zusammensetzung nach Anspruch 5, dadurch gekennzeichnet, daß das ein fluoraliphatisches Radikal enthaltende Poly(oxyalkylen) die allgemeine Formel

$$(R_f)_sZ(R^3)_yZ'B_t \text{ oder } (R_f)_sZ(R^3)_yZ'B'_{t\ w}$$

hat, in der

$R_f$ das genannte fluoraliphatische Radikal ist,

Z eine Verbindung ist, die $R_f$ und $(R^3)y$ kovalent miteinander verbindet,

$(R^3)_y$ ein Poly(oxyalkylen)anteil ist, wobei $R^3$ ein Oxyalkylen mit 2 bis 4 Kohlenstoffatomen ist und y eine ganze Zahl oder eine Zahl mit mindestens dem Wert 5 ist und einen Wert von bis zu 100 oder mehr haben kann,

B ein endständiges einwertiges organisches Radikal ist,

B' B oder eine Valenzbindung ist, wobei mindestens ein B' eine Valenzbindung ist, die ein mit Z verbundenes Radikal $(R^3)_y$ mit einem anderen Z verbindet,

Z' eine Verbindung ist, die B oder B' und $(R^3)y$ kovalent miteinander verbindet,

s eine ganze Zahl oder eine Zahl mit mindestens dem Wert 1 ist und einen Wert von bis zu 25 oder mehr haben kann,

t eine ganze Zahl oder eine Zahl mit mindestens dem Wert 1 ist und einen Wert von bis zu 60 oder mehr haben kann und

w eine ganze Zahl oder eine Zahl mit mindestens dem Wert 1 ist und einen Wert von bis zu 30 oder mehr haben kann.

7. Zusammensetzung nach Anspruch 5, dadurch gekennzeichnet, daß das fluorochemische Poly(oxyalkylen) das Copolymer von

$C_8F_{17}SO_2N(C_4H_9)C_2H_4O_2CCH=CH_2$, $CH_2=CHCO_2(C_2H_4O)_{10}(C_3H_6O)_{22}(C_2H_4)_9C_2H_4O_2CCH=CH_2$, und $CH_2=CHCO_2(C_2H_4O)_{10}(C_3H_6O)_{22}(C_2H_4)_9C_2H_4OH$ ist.

8. Zusammensetzung nach Anspruch 5, dadurch gekennzeichnet, daß das fluorchemische Guanidin durch die Formel

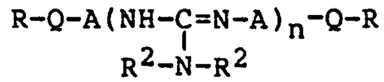 IV

dargestellt ist, in der R—Q $C_8F_{17}SO_2N(C_2H_5)C_2H_4OCONH$— ist, A —$C_6H_4CH_2C_6H_4$— oder —$CH_2C_6H_4CH_2$— ist, $R^2$—N—$R^2$ is $N(C_4H_9)_2$, — 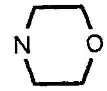 , oder —$N(iso\text{-}C_3H_7)_2$ ist und n gleich 2 ist und dab das fluorochemische Poly(oxyalkylen) das Copolymer von

$C_8F_{17}SO_2N(C_4H_9)C_2H_4O_2CCH{=}CH_2$, $CH_2{=}CHCO_2(C_2H_4O)_{10}(C_3H_6O)_{22}(C_2H_4)_9C_2H_4O_2CCH{=}CH_2$, und $CH_2{=}CHCO_2(C_2H_4O)_{10}(C_3H_6O)_{22}(C_2H_4)_9C_2H_4OH$ ist.

9. Faserappretur, die wenigstens teilweise aus einer organischen Lösung oder einer wäßrigen Dispersion einer fluorchemischen Guanidinverbindung nach einem der vorhergehenden Ansprüche besteht.

10. Faserappretur nach Anspruch 9, die außerdem einen Gleitstoff für die Fasern enthält.

11. Verfahren zum Öl- und Wasserabweisendmachen eines Fasermaterialsubstrats, dadurch gekennzeichnet, daß die Oberfläche des Sustrats mit einer Faserappretur nach Anspruch 9 oder 10 behandelt wird.

12. Verfahren zum Behandeln von gesponnenen organischen Chemiefasern, dadurch gekennzeichnet, daß auf die Fasern eine Faserappretur nach Anspruch 9 oder 10 aufgetragen wird.

13. Fasermaterialsubstrat, das mit einer fluorchemischen Guanidinverbindung nach einem der Ansprüche 1 bis 8 überzogen ist.

14. Überzogenes Fasermaterialsubstrat nach Anspruch 13, dadurch gekennzeichnet, daß das Substrat aus Nylonteppischfasern besteht.

**Revendications**

1. Dérivé de guanidine insoluble dans l'eau, habituellement solide, répondant à la formule générale:

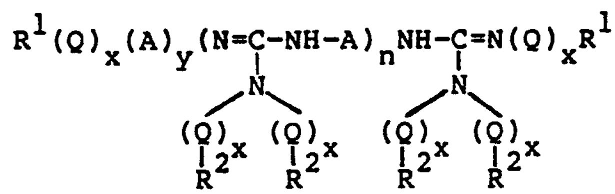

et renfermant un ou plusieurs radicaux fluoroaliphatiques monovalents, $R_f$, qui sont à la fois oléophobes et hydrophobes, fluorés, stables, inertes et non polaires et qui possèdent 3 à 20 atomes de carbon et au moins trois atomes de carbone totalement fluorés, contenant 40 à 78% en poids de fluor, formule dans laquelle chaque indice x possède indépendamment la valuer 0 ou 1, y est égal à 0 ou 1; n a une valeur de 0 à 20; $R^1$ et $R^2$ représentent des atomes d'hydrogène, ledit radical $R_f$ ou un autre radical organique contenant 1 à 18 atomes de carbone choisis entre des groupes alkyle, cycloalkyle, aryle et leurs associations pouvant contenir des groupements renfermant des hétéroatomes et qui sont dépourvus d'atomes d'hydrogène actif, mais sous réserve qu'au moins l'un des substituants $R^1$ et $R^2$ représente ledit substituant $R_f$ et au moins l'un des substituants $R^1$ et $R^2$ représente l'autre radical organique, les deux groupes $R^2$ d'un groupement guanidino,

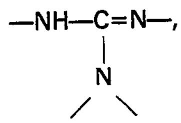

étant facultativement liés l'un à l'autre pour former une structure cyclique renfermant l'atome d'azote latéral,

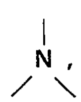

du groupement guanidino; A représente un groupe organique de jonction divalent contenant 2 à 20 atomes de carbone choisi entre des groupes alkylène, das groupes aralkylène, das groupes arylène, des groupes polyoxyalkylène, et leurs associations, pouvant contenir des groupes renfermant des hétéoatomes, qui est dépourvu d'atomes d'hydrogène actif et qui peut contenir facultativement ledit groupe fluoroaliphatique $R_f$; Q représente un groupe de jonction divalent contenant des hétérotomes ou organique, ou une association de tels groupes, choisis entre des groupes aliphatiques polyvalents, des groupes aromatiques

polyvalents, des groupes oxy, thio, carbonyle, sulfone, sulfoxy, $—N(CH_3)—$, sulfonamido, carbonamido, sulfonamidoalkylène, carbonamidoalkylène, carbonyloxy, uréthanne et urée, et qui est dépourvu d'atomes d'hydrogène actif; sous réserve que, lorsqu'un seul groupement guanidino est présent et lorsque seulement deux substituants organiques, $—(Q)_xR^1$ ou $—(Q)_xR^2$, sont présents dans ledit groupement guanidino, lesdits substituants soient sur des atomes d'azote différents et ledit composé contienne 20 à 70% en poids de fluor lié au carbone.

2. Dérivé fluoré de guanidine suivant la revendication 1, représenté par la formule

$$R\text{-}Q\text{-}A(NH\text{-}\underset{R^2\text{-}N\text{-}R^2}{\underset{|}{C}}=N\text{-}A)_n\text{-}Q\text{-}R$$

dans laquelle R—Q représente un groupe $C_8F_{17}SO_2N(C_2H_5)C_2H_4OCONH—$, A représente un groupe $—C_6H_4CH_2C_6H_4—$ ou $—CH_2C_6H_4CH_2—$, $R^2—N—R^2$ représente un groupe $—N(C_4H_9)_2$ et n est égal à 2.

3. Dérivé fluoré de guanidine suivant la revendication 1, représenté par la formule

$$R\text{-}Q\text{-}A(NH\text{-}\underset{R^2\text{-}N\text{-}R^2}{\underset{|}{C}}=N\text{-}A)_n\text{-}Q\text{-}R$$

dans laquelle R—Q représente un groupe $C_8F_{17}SO_2N(C_2H_5)C_2H_4OCONH—$, A représente un groupe $—C_6H_4CH_2C_6H_4—$ ou $—CH_2C_6H_4CH_2—$, $R^2—N—R^2$ représente un groupe — 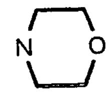 , et n a une valeur de 1 à 21.

4. Dérivé fluoré de guanidine suivant la revendication 1, représenté pa la formule

$$R\text{-}Q\text{-}A(NH\text{-}\underset{R^2\text{-}N\text{-}R^2}{\underset{|}{C}}=N\text{-}A)_n\text{-}Q\text{-}R$$

dans laquelle R—Q représente un groupe $C_8F_{17}SO_2N(C_2H_5)C_2H_4OCONH—$, A represéte un groupe $—C_6H_4CH_2C_6H_4—$ ou $—CH_2C_6H_4CH_2—$, $R^2—N—R^2$ représente un groupe $—N(iso—C_3H_7)_2$ et n a une valeur de 1 à 21.

5. Composition comprenant un mélange: (a) d'un dérivé fluoré de guanidine suivant l'une quelconque des revendications 1 à 4, et (b) d'un poly(oxyalkylène) habituellement liquide ou solide à bas point de fusion, soluble ou dispersable dans l'eau, contenant un radical fluoro-aliphatique, ou d'une composition comprenant un mélange de ces poly(oxyalkylènes), ledit poly(oxyalkylène) comprenant ou un plusieurs desdits radicaux fluoroaliphatiques et un ou plusieurs groupes poly(oxyalkylènes), lesdits radicaux et groupements poly(oxyalkylène) étant liés les uns aux autre par des groupes contenant des hétéroatomes ou des groupes organiques de jonction, ou bien des associations de ces groupes.

6. Composition suivant la revendication 5, dans laquelle le poly(oxyalkylène) contenant un radical fluoroaliphatique répond à la formule générale:

$$(R_f)_sZ[(R^3)_yZ'B]_t \text{ ou } [(R_f)_sZ[(R^3)_yZ'B']_t]_w$$

dans laquelle

$R_f$ représente ledit radical fluoroaliphatique,

Z représente une jonction reliant l'un à l'autre par covalence $R_f$ et $(R^3)_y$,

$(R^3)_y$ représente un groupe poly(oxyalkylène), $R_3$ étant un groupe oxyalkylène de 2 à 4 atomes de carbone et y étant un nombre entier ou un nombre au moins égal à 5 et pouvant atteindre une valeur égale ou supérieure à 100,

B représente un radical organique monovalent terminal,

B' représente le radical B ou une liaison de covalence, sous réserve qu'au moins un groupe B' représente une liaison de valence permettant l'interconnexion d'un radical $(R^3)_y$ lié à Z à une autre jonction Z,

Z' représente une jonction reliant l'un à l'autre par covalence B ou B' et $(R^3)_y$,

s est un nombre entier ou un nombre au moins égal à 1 et pouvant être égal ou supérieur à 25,

t est un nombre entier ou un nombre au moins égal à 1 et pouvant être égal ou supérieur à 60, et

w est un nombre entier ou un nombre supérieur à 1 et pouvant être égal ou supérieur à 30.

7. Composition suivant la revendication 5, dans laquelle le dérivé fluoré de poly(oxyalkylène) est le copolymère de

$C_8F_{17}SO_2N(C_4H_9)C_2H_4O_2CCH=CH_2$, $CH_2=CHCO_2(C_2H_4O)_{10}(C_3H_6O)_{22}(C_2H_4)_9C_2H_4O_2CCH=CH_2$, et $CH_2=CHCO_2(C_2H_4O)_{10}(C_3H_6O)_{22}(C_2H_4)_9C_2H_4OH$.

8. Composition suivant la revendication 5, dans laquelle le dérivé fluoré de guanidine est représenté par la formule

$$R-Q-A(NH-\underset{R^2-N-R^2}{\underset{|}{C}}=N-A)_n-Q-R$$

dans laquelle R—Q représente un groupe $C_8F_{17}SO_2N(C_2H_5)C_2H_4OCONH—$, A représente un groupe $—C_6H_4CH_2C_6H_4—$ ou $—CH_2C_6H_4CH_2—$, $R^2—N—R^2$ represénte un groupe $—N(C_4H_9)_2$, — 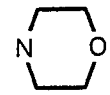 , ou $—N(iso\text{-}C_3H_7)_2$ et n est égal à 2 et dans laquelle le dérivé fluoré de poly(oxyalkylène) est le copolymère de $C_8F_{17}SO_2N(C_4H_9)C_2H_4O_2CCH=CH_2$, $CH_2=CHCO_2(C_2H_4O)_{10}(C_3H_6O)_{22}(C_2H_4)_9C_2H_4O_2CCH=CH_2$, et $CH_2=CHCO_2(C_2H_4O)_{10}(C_3H_6O)_{22}(C_2H_4O)_9C_2H_4OH$.

9. Apprêt pour fibres, comprenant une solution organique ou une dispersion aqueuse d'un dérivé fluoré de guanidine suivant l'une quelconque des revendications précédentes.

10. Apprêt pour fibres suivant la revendication 9, comprenant en outre un lubrifiant de fibres.

11. Procédé pour conférer un pouvoir oléofuge et hydrofuge à un substrat fibreux, qui consiste à traiter la surface de ce substrat avec un apprêt pour fibres suivant la revendication 9 ou la revendication 10.

12. Procédé de traitement de fibres organiques synthétiques filées, dans lequel un apprêt pour fibres suivant la revendication 9 ou la revendication 10 est appliqué auxdites fibres.

13. Substrat fibreux revêtu avec un dérivé fluoré de guanidine suivant l'une quelconque des revendications 1 à 8.

14. Substrat fibreux revêtu suivant la revendication 13, dans lequel le substrat est une fibre de Nylon pour tapis.